**Europäisches Patentamt**

(19) **European Patent Office**

**Office européen des brevets**

(11) Publication number : **0 464 220 B1**

(12) # EUROPEAN PATENT SPECIFICATION

(45) Date of publication of patent specification :
**27.09.95 Bulletin 95/39**

(51) Int. Cl.$^6$ : **H04R 1/10, G10K 11/16**

(21) Application number : **91902743.3**

(22) Date of filing : **18.01.91**

(86) International application number :
**PCT/JP91/00058**

(87) International publication number :
**WO 91/11078 25.07.91 Gazette 91/17**

(54) **EARPHONE DEVICE.**

(30) Priority : **19.01.90 JP 8509/90**
       **19.01.90 JP 8510/90**

(43) Date of publication of application :
**08.01.92 Bulletin 92/02**

(45) Publication of the grant of the patent :
**27.09.95 Bulletin 95/39**

(84) Designated Contracting States :
**DE FR GB NL**

(56) References cited :
**EP-A- 0 360 517**
**WO-A-89/00746**
**JP-A- 6 180 997**
**JP-A-60 220 698**
**JP-U- 0 609 397**
**JP-U- 5 756 083**
**JP-U-55 165 574**

(73) Proprietor : **SONY CORPORATION**
**7-35 Kitashinagawa 6-chome**
**Shinagawa-ku**
**Tokyo 141 (JP)**

(72) Inventor : **INANAGA, Kiyofumi Sony**
**Corporation**
**7-35, Kitashinagawa 6-chome**
**Shinagawa-ku Tokyo 141 (JP)**
Inventor : **SOGAWA, Hiroyuki Sony**
**Corporation**
**7-35, Kitashinagawa 6-chome**
**Shinagawa-ku Tokyo 141 (JP)**
Inventor : **IIDA, Yasuhiro Sony Corporation**
**7-35, Kitashinagawa 6-chome**
**Shinagawa-ku Tokyo 141 (JP)**
Inventor : **KIMURA, Akira Sony Corporation**
**7-35, Kitashinagawa 6-chome**
**Shinagawa-ku Tokyo 141 (JP)**

(74) Representative : **Körber, Wolfhart, Dr.rer.nat.**
**et al**
**Patentanwälte Mitscherlich & Partner**
**Postfach 33 06 09**
**D-80066 München (DE)**

## Description

## TECHNICAL FIELD

The present invention relates to a so-called active type earphone device which is capable of reducing external noises.

## BACKGROUND TECHNIQUE

So-called ear fitting type noise reducing apparatus which is used in working in an environment in which external noises are very loud has been widely known. This ear fitting type noise reducing apparatus is worn in such a manner that the headphone cap covers the ears for reducing the external noises. The headphone cap is pressed upon the temple of the head so that external noises from the environment will not pass through a gap between the temple of the head and the headphone cap. The noise reducing apparatus has been used in such a manner.

However, it is necessary to strongly press the headphone cap of the above mentioned prior art noise reducing apparatus upon the temple of the head in order to prevent external noises from the environment from entering the gap between the headphone cap and the temple of the head. Accordingly, an oppressive sensation is felt on the temple of the head and the noise reducing apparatus per se is large in size and heavy. Therefore, use of this apparatus for a long period of time is unbearable. Only a headphone cap cannot shield noise having low frequencies (several hundred Hz to not higher than 1 KHz). If noises are reduced by the above mentioned method and reproduced sounds are tried to heared, an effect of so-called localization in head occurs due to reflections of the reproduced sound in the headphone cap so that an adverse influence is given to communication, etc. The effect of localization in head occurs as follows: Reflections occur between the headcap and the entrance of the external auditory canal since the acoustic impedance in the entrance of the external auditory canal is different from the impedance in the external auditory canal. The reflected wave returns to the ear drum, resulting in that a sound image is localized in the head. Uncomfortable feeling as if the ear were plugged is felt.

So-called active type earphone devices have been known as earphone devices used in working in external loud noise place. Such type earphone devices reduce the noises generated near the headphone unit by converting the external noises into electrical signals by a microphone unit and by negatively feeding back the converted electrical signal in an opposite phase via a negative feed back loop.

Such active type earphone device has a high sound shielding ability and is thus capable of almost shielding external sounds. Accordingly, it is very inconvenient for the user to remove the earphone device from the ear each time when the user desires to listen to external sounds such as emergency information or human voices.

Such active type earphone device operating as acitve noise reduction system is known from WO 89/00746. This device comprises an internal microphone unit disposed in the vicinity of the ear, the pickup signal of which is supplied via a feedback loop to an internal microphone unit supplying an inverted signal of the signal picked up by the internal microphone. There are further described loop stabilization means and high pass frequency filter means to improve the phase stability of the feedback loop especially in the presence of high pressure sound pulses occuring for example in military applications.

The present invention was made under such circumstance.

It is an object of the present invention to provide an earphone device which will not cause an effect of localization in head and can be used for a long period of time without pressing the head and is capable of effectively reducing external noises.

It is another object of the present invention to provide an earphone device with which external sound can be listened even if the earphone device which has been worn is not removed.

## DISCLOSURE OF THE INVENTION

An earphone device of the present invention comprises an acoustic tube having an inner diameter which is substantially identical with that of the external auditory canal and is provided with an ear mounting portion at one end thereof and with an acoustic non-reflecting terminal at the other end thereof; and an earphone unit and an internal microphone unit which are disposed in the vicinity of the ear when the device is worn and are mounted on the periphery of the acoustic tube in adjacent relationship with each other so that vibration plates of the units face inwardly of the tube, whereby signal obtained by the inner microphone unit, the phase of which has been reversed is supplied to the earphone unit for reducing the external noises. In the earphone device of the present invention, external noises are effectively reduced without pressing the temple of the head and uncomfortable feeling such as oppression is not felt even if the earphone device is used for a long period of time.

An earphone device of the present invention comprises an acoustic tube having an inner diameter which is substantially identical with that of the external auditory canal and is provided with an ear mounting portion at one end thereof and with an acoustic non-reflecting terminal at the other end thereof; an external microphone unit disposed externally of said acoustic tube for converting the external sound into an electrical signal; an earphone unit and an internal microphone unit which are mounted on the periphery of the acoustic tube in adjacent relationship with each other so that vibration plates of the units face inwardly of the tube; and mixing ratio variable means for mixing the input audio signal, the signal obtained by phase reversion of the signal obtained from said internal microphone unit and the signal obtained from the external microphone unit with each other at a desired ratio, the output signal of said mixing means being supplied to said earphone unit. In the earphone device of the present invention, external sounds can be listened to while the device is worn on the head and external noises which reach the ear can be reduced.

## BRIEF DESCRIPTION OF THE DRAWINGS

Fig. 1 is a sectional view of a part of an acoustic tube of an earphone device of the present invention;
Fig. 2 is a block diagram showing the structure of the earphone device;
Fig. 3 is a perspective view showing the appearance of a head set to which the earphone device is applied; and
Fig. 4 is a block diagram showing another structure of the earphone device of the present invention.

## BEST MODES FOR EMBODYING THE INVENTION

Embodiments of the earphone of the present invention will be described with reference to the drawings.

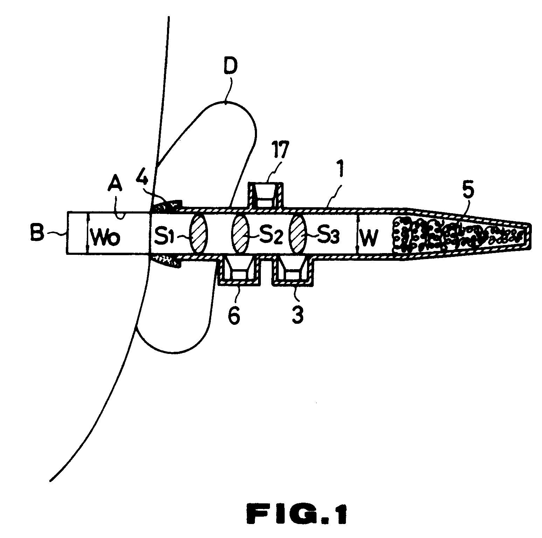

Fig. 1 is a sectional view of an earphone device of the present invention.

In Fig. 1, the earphone device comprises an acoustic tube 1 having an inner diameter W which is substantially identical with the inner diameter $W_0$ of the external auditory canal A and which is provided with auricle mounting member 4 made of a material such as synthetic resin or rubber having an elasticity at one end thereof and with a sound absorbing member 5 such as felt at the other end thereof so that the other end becomes an acoustic non-reflection terminal, an earphone unit 3 and an internal microphone unit 6 adjacent to the earphone unit 3 provided on the periphery of the acoustic tube 1, each having a vibration plate facing inward of the tube.

The acoustic tube 1 forms an earphone casing. The inner diameter W is made uniform and substantially identical with the inner diameter $W_0$ of the external auditory canal A and the sound absorbing member 5 is provided at the other end to provide the acoustic non-reflecting terminal so that the acoustic impedance of the acoustic tube 1 is made substantially identical with the impedance of the external auditory canal A. So called localization in head is thus prevented. In order to prevent a change in the acoustic impedance, the earphone unit 3 and the inner microphone unit 6 are mounted on the side of the acoustic tube 1 so that the respective plates are in parallel relationship and the sectional area $S_1$ of the acoustic tube 1 is equal to those $S_2$ and $S_3$ of the mounting portions of the earphone unit 3 and the microphone unit 6.

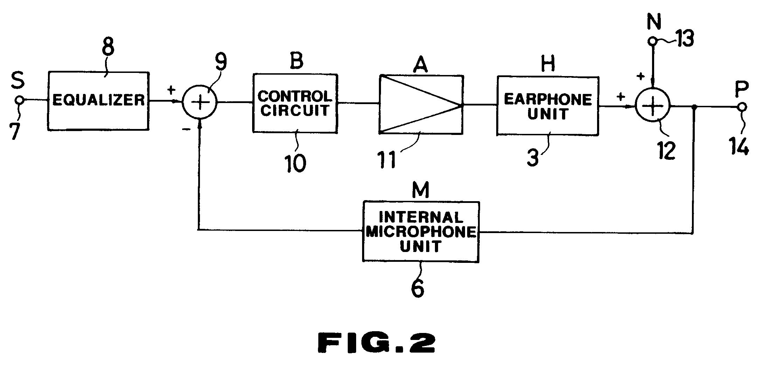

Fig. 2 is a block diagram showing the structure of the earphone device.

In this earphone device, a received signal is supplied to an equalizer 8 via an input terminal 7 as an input audio signal as shown in Fig. 2.

The equalizer 8 puts an emphasis upon the sound components in the input audio signal by increasing the amplification in an intermediate range of the frequency of the input audio signal. The output signal is supplied to a control circuit 10 via an adder 9 which will be described hereafter. Adjustment of the frequency in the equalizer 8 may be achieved by a desired level by adjustment.

The control circuit 10 controls the frequency characteristics of an input signal supplied from the adder 9 so that frequency characteristics of the whole circuit from the equalizer 8 to the earphone unit 3 corresponds to the audio frequency characteristics until the external noises reaches the ear.

An output signal from the control circuit 10 is supplied to the earphone unit 3 via an amplifier 11. The amplifier may change its amplification factor in order to make easier to listen to reproduced sounds.

The earphone unit 3 provides a reproduced sound by electro-acoustically converting the output signal supplied from the amplifier 11.

The adder 12 adds the reproduced sound reaching the external auditory canal A after provided from the earphone unit 3 with external noises supplied from the input terminal 13, that is, the external noises reaching the external auditory canal A via the earphone unit 3 and the acoustic tube 1 and external noises reaching the external auditory canal A through a gap between the earphone unit 3 and the ear. The adder 12 represents this operation in equivalent block diagram. The reproduced sound which has been added with the external noises is picked up by the internal microphone 6 and is converted into an electrical signal. The internal microphone unit 6 reverses the phase of the electrical signal representative of the picked-up reproduced sound for supplying the same to the adder 9.

The adder 9 adds a signal having an reversed phase supplied from the internal microphone unit 6 with the output signal from the equalizer 8.

This enables the sound provided from the earphone unit 3 to become a clear reproduced sound from which external noises are removed.

The reproduced sound is outputted via the output terminal 14.

If the level of the signal supplied from the input terminal 7, the transfer functions of the internal microphone unit 6, of the control circuit 10, of the amplifier circuit 11, and of the earphone unit 3, the level of the external noises supplied from the input terminal 13 and the level of the reproduced sound output from the output terminal 14 are represented as S, M, $\beta$, A, H, N and P, respectively, the level P of the reproduced sound is represented as follows:

$$P = \frac{AHM\beta}{1 + AHM\beta} S + \frac{1}{1 + AHM\beta} N$$

The transfer functions M, $\beta$, A and H are represented in frequency domain. If $AHM\beta >> 1$ at this time, the level P of the reproduced sound is represented as follows:

$$P = S + \frac{1}{AHM\beta} N$$

It is found from the above equation that the level P of the reproduced sound does not depend the transfer functions A and B of the amplifier circuit 11 and the earphone unit 3 if the condition $AHM\beta >> 1$ is satisfied. Accordingly, the level of the external noise which has been phase reversed by the internal microphone unit 6 independently of the level of the reproduced sound by changing the gain of the amplifier 11 under a condition $AHM\beta >> 1$. The effect of localization in head can be effectively prevented so that the reproduced sound can be clearly listened by increasing the gain of the amplifier circuit 11 to increase the reduction amount of noise when the external noise is loud and by decreasing the gain of the amplifier circuit 11 when the external noise is less loud.

There is the possibility that the external sound can never be listened if the noise reduction amount is increased by increasing the gain of the amplifier circuit 11. In order to enable the external sound to be listened, an external microphone 17 for picking up external sounds may be provided externally of the acoustic tube 1. The signal of the external sound provided by the external microphone 17 may be supplied to the adder 9 in which it is added with the input audio signal.

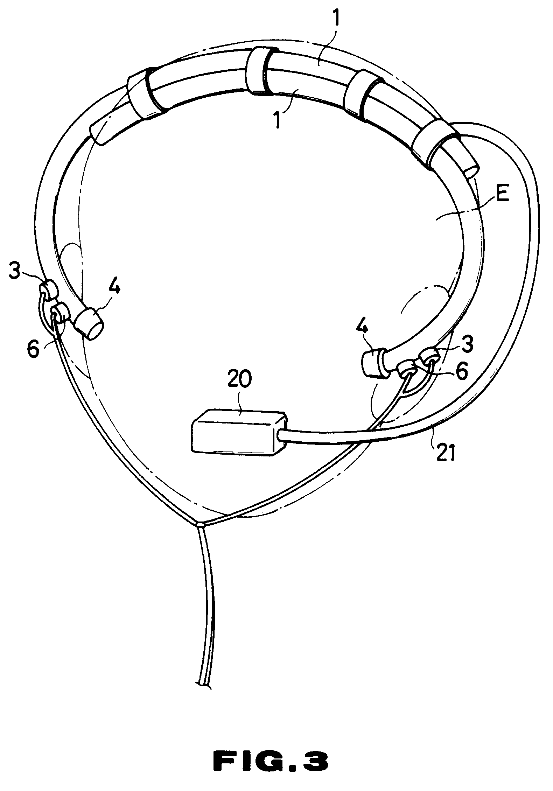

Fig. 3 is a perspective view showing the appearance of a head set to which the present earphone device is applied.

In the head set shown in Fig. 3, the acoustic tube 1 is bent by such an angle that the acoustic impedance is not influenced. Two acoustic tubes 1 and 1 are linked with each other by a link member 15 so that the respective mounting members 4 face to inlets of the auditory canals. One end of a bar 21 is mounted on, for example, the link member 15 and a transmitting microphone 20 is mounted on the other end of the bar 21. Two acoustic tubes 1 overhang the head E as a hair band.

If the mounting members 4 are mounted on the auricles D while the mounting members 4 of the acoustic tubes 1 are slightly opened, the mounting members 4 would be biased upon the entrances of the external auditory canals A on the both sides in such a manner that they will not press the temples of the head. The mounting members 4 are prevented from being removed from the auricles D. Comfortable wearing and clear communication can be achieved.

It is apparent from the foregoing that there is no reflections of sounds in the tubes and no effect of localization in head occurs and external noises can be effectively reduced and a compact and light-weight earphone device can be provided. Therefore, sounds, etc. can be clearly listened even if a high volume is not reproduced so that sounds from surroundings cannot be failed to listen. Sound distortion in the earphone unit can be reduced by the negative feedback.

If the earphone device is used as, for example, a head set, it is not necessary to cover and press the ears with headphone caps. Accordingly, fatigue due to use for a long period of time can be reduced.

The earphone device of the present invention may be, of course, used for, for example, earphone devices of compact portable headphone players and the like and handsets of telephones.

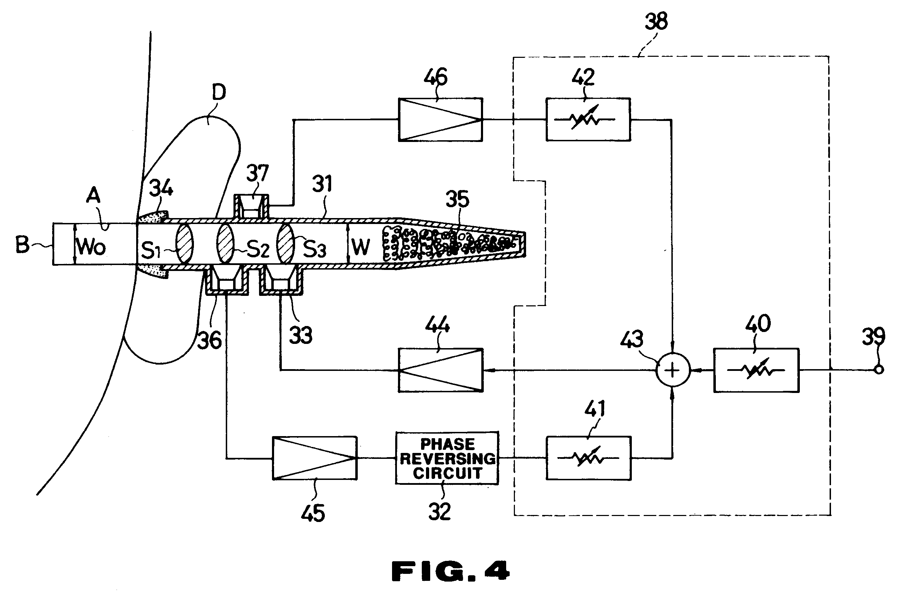

Another embodiment of the earphone device of the present invention will be described with reference to Fig. 4.

In Fig. 4, the earphone device is formed so that it has an inner diameter W which is substantially identical with the inner diameter $W_0$ of the external auditory canal A.

The earphone device comprises an acoustic tube 31 having an inner diameter W which is substantially identical with the inner diameter $W_0$ of the external auditory canal A and which is provided with auricle mounting member 34 made of a material such as synthetic resin or rubber having an elasticity at one end thereof and with a sound absorbing member 35 such as felt at the other end thereof so that the other end becomes an acoustic non-reflection terminal, an earphone unit 33, an internal microphone unit 36 adjacent to the earphone unit 33 provided on the periphery of the acoustic tube 1, each having a vibration plate facing inward of the tube, a phase reversing circuit 32 which reverses the phase of an audio signal in the acoustic tube 31 supplied from the internal microphone unit 36 for outputting it as a phase reversed signal, an external microphone unit 37 disposed externally of the acoustic tube 31, which picks up external noises for outputting an external noise signal and a mixing circuit 38 which is capable of mixing the inputted audio signal such as music signal reproduced from a compact portable stereo cassette tape recorder with the phase reversed signal and the external noise signal at a desired mixing ratio.

The mixing circuit 38 comprises a first variable gain control circuit 40 for variably changing the gain of the inputted audio signal, a second variable gain control circuit 41 for variably changing the gain of the phase reversed signal, a third variable gain control circuit 42 for variably changing the gain of the external noise signal and an adder 43 for adding the output signals from the variable gain control circuits 40, 41 and 42.

The acoustic tube 31 forms an earphone casing. The inner diameter $W_0$ of the external auditory canal A and the sound absorbing member 35 is provided at the other end to provide the acoustic non-reflecting terminal so that the acoustic impedance of the acoustic tube 31 is made substantially identical with the impedance of the external auditory canal A. So called localization in head is thus prevented. In order to prevent a change in the acoustic impedance, the earphone unit 33 and the inner microphone unit 36 are mounted on the side of the acoustic tube 31 so that the respective plates are in parallel relationship and the section area $S_1$ of the acoustic tube 31 is equal to those $S_2$ and $S_3$ of the mounting portions of the earphone unit 33 and the microphone unit 36.

Now, operation of the earphone device will be described.

Input audio signal such as music signal which has been reproduced from a compact portable stereo cassette tape recorder is supplied from an input circuit 39 to an amplification circuit 44 via the first variable gain control circuit 40 and the adder 43 of the mixing circuit 38 and is amplified by the amplification circuit 44 and is supplied to the earphone unit 33.

The earphone unit 33 electro-acoustically converts the output signal from the amplification circuit 41 for providing reproduced sound.

The sound provided by the earphone unit 33 transmits to the eardrum via the external auditory canal A and is picked up by the internal microphone unit 36. The sound which is picked up by the internal microphone unit 36 is supplied as audio signal to the phase reversing circuit 32 via the amplification circuit 45.

The phase reversing circuit 32 reverses the phase of the audio signal supplied from the amplification circuit 45. The output signal from the phase reversing circuit 32 is supplied as signal having a phase opposite to that of the audio signal to the adder 43 via the second variable gain control circuit 41.

On the other hand, the external microphone unit 37 picks up the external noise transmitting through the auditory canal A. The external noise signal obtained by picking up the external noise by the external microphone unit 37 is amplified by the amplification circuit 46 and is supplied to the adder 43 via the third variable gain control circuit 42.

The adder 43 adds the inputted audio signal, the negatively fed back signal, and the external noise signal with each other. The added signal is supplied to the earphone unit 33 via the amplification circuit 44.

The earphone unit 33 converts the output signals supplied from the amplification circuit 44 into audio signal for providing sounds.

The earphone device of this embodiment is capable of controlling the mixing ratio of the input audio signal, the negatively fed back signal and the external noise signal which are added with each other in the adder 33 as mentioned above by means of the variable gain control circuits 40, 41 and 42.

That is, on normal use in which the reproduced sound of the input audio signal is desired to be listened without being influenced by the external noise, the gain of the external noise signal is adjusted to a low value or zero by the third variable gain control circuit 42. The gains of the input audio signal and the negatively fed back signal are adjusted to desired magnitudes by the first and second variable gain control circuits 40 and 41. Therefore, excellent reproduced sound having less noises can be listened.

Conversely, when external emergency information and human voices are desired to be listened while listening to such reproduced sounds, the gains of the input audio signal and the negatively fed back signal are not adjusted or adjusted to low values or zero and the gain of the external noise is increased by adjusting third variable gain control circuit 42, the gain of which has been a low value or zero. The external emergency information and the human voices can be listened while the earphone device is worn, that is, without removing the earphone device from the ear.

The earphone device can be used as a so-called ear plug by turning off the first variable gain control circuit 40 so that the external noises reaching at the eardrum B are canceled by the negative feed back loop to provide a substantially silent condition. Also in this case, external sounds can be listened while the earphone device is worn by adjusting the gain of the external noise signal by the third variable gain control circuit 42.

Adjustment of the gains of the variable gain control circuits 40, 41 and 42 can be achieved by operation of switches or knobs.

## Claims

1. An earphone device comprising
   an acoustic tube (1) having an inner diameter (w) which is substantially identical to that of the external auditory canal (A) and is provided with an ear mounting portion (4) at one end thereof and with an acoustic non-reflecting terminal (5) at the other end thereof; and
   an earphone unit (3) and an internal microphone unit (6) which are disposed in the vicinity of the ear when the device is worn and are mounted on the periphery of the acoustic tube (1) in adjacent relationship with each other so that vibration plates of the units (3, 6) face inwardly of the tube (1), whereby a signal obtained by the inner microphone unit (6), the phase of which has been reversed is supplied to the earphone unit (3) for reducing external noises.

2. An earphone device as defined in claim 1 and further including an adder (9) for adding the signal having a phase opposite to that of the signal obtained from said inner microphone unit (6) with input audio signal;
a control circuit (10) for controlling the frequency characteristics of output signal of said adder (9); and
an amplification circuit (11) for amplifying the output of said control circuit (10) to supply the amplified output to said earphone unit (3); whereby the transfer functions M, β, A and H of said internal microphone unit (6), said control circuit (10), said amplification circuit (11) and said earphone unit (3) which are represented in the frequency domain are set so that a condition AHMβ > > 1 is satisfied.

3. An earphone device as defined in claim 2 in which the transfer function A of said amplification circuit (11) is made variable so that a condition AHMβ > > 1 is satisfied.

4. An earphone device as defined in claim 1 in which an external microphone unit (17) for converting external sound into an electrical signal is provided externally of said acoustic tube (1), and in which the input audio signal, the signal having a phase opposite to that of the signal obtained from said internal microphone unit (6), the signals obtained by said external (6) and internal (17) microphone units are mixed with each other by mixing means and the mixed signal is supplied to said earphone unit (3).

5. An earphone device comprising an acoustic tube (31) having an inner diameter (w) which is substantially identical to that of the external auditory canal (A) and is provided with an ear mounting portion (34) at one end thereof and with an acoustic non-reflecting terminal (35) at the other end thereof;
an external microphone unit (37) disposed externally of said acoustic tube (31) for converting external sound into an electrical signal;
an earphone unit (33) and an internal microphone unit (36) which are mounted on the periphery of the acoustic tube (31) in adjacent relationship with each other so that vibration plates of the units face inwardly of the tube (31); and
variable mixing ratio means (38) for mixing the input audio signal, the signal obtained by phase reversion of the signal obtained from said internal microphone unit (36) and the signal obtained from the external microphone unit (37) with each at a desired ratio, the output signal of said mixing means (38) being supplied to said earphone unit (33).

6. An earphone unit as defined in claim 5 in which said mixing means (38) includes a first variable gain control circuit (40) for variably changing the gain of the input audio signal;
a second variable gain control circuit (41) for variably changing the gain of the signal obtained by phase reversion of the signal obtained from said internal microphone unit (36);
a third variable gain control circuit (42) for variably changing the gain of the signal obtained from said external microphone unit (37); and
an adder (43) for adding respective outputs from the first to third variable gain control circuits (40, 41, 42).

7. An earphone device as defined in claim 5 in which said earphone unit (33) and internal microphone unit (36) are mounted on the side of said acoustic tube (31) so that the respective vibration plates are in parallel.

8. An earphone device as defined in claim 7 in which the sides of said vibration plates of said earphone unit (33) and internal microphone unit (36) are flush with the inner surface of said acoustic tube (31).

9. An earphone device as defined in claim 8 in which said acoustic tube (31) has an inner diameter which is substantially constant from said auricle mounting portion (34) to said non-reflection terminal (35).

**Patentansprüche**

1. Ohrhörervorrichtung mit
einem Akustik-Röhrchen (1), das einen Innendurchmesser (w) aufweist, der im wesentlichen mit dem äußeren Gehörgang (A) identisch ist, und das an einem Ende einen Ohr-Anschlußabschnitt (4) aufweist und am anderen Ende mit einem nichtreflektierenden Akustik-Anschluß (5) versehen ist, und mit
einer Ohrhörereinheit (3) und einer inneren Mikrophoneinheit (6), die in der Nähe des Ohres angeordnet sind, wenn die Vorrichtung getragen wird, und die am Umfang des Akustik-Röhrchens (1) zueinander naheliegend angeordnet sind, so daß die Vibrationsscheiben der Einheiten (3, 6) ins Innere des Röhrchens (1) gerichtet sind, wodurch mittels der internen Mikrophoneinheit (6) ein Signal, dessen Phase umgekehrt ist, erhalten wird, das der Ohrhörereinheit (3) zum Vermindern externer Geräusche zugeführt wird.

2. Ohrhörervorrichtung nach Anspruch 1, die weiterhin, um dem Eingangs-Audiosignal das Signal zu addieren, das eine Phase aufweist, die dem von der inneren Mikrophoneinheit (6) erhaltenen Signal entgegengesetzt ist, einen Addierer (9) zum Steuern des Frequenzganges des Ausgangssignals des Addierers (9) eine Steuerschaltung (10), und eine Verstärkerschaltung (11) aufweist, um das Ausgangssignal der Steuerschaltung (10) zu verstärken, um das verstärkte Ausgangssignal der Ohrhörereinheit (3) zuzuführen, wodurch die Übertragungsfunktionen M, β, A und H der inneren Mikrophoneinheit (6), der Steuerschaltung (10), Verstärkerschaltung (11) und der Ohrhörereinheit (3), die im Frequenzbereich angegeben sind, so eingestellt sind, daß eine Bedingung AHMβ > > 1 erfüllt ist.

3. Ohrhörervorrichtung nach Anspruch 2, bei dem die Übertragungsfunktion A der Verstärkerschaltung (11) veränderbar ist, so daß einer Bedingung AHMβ > > 1 erfüllt ist.

4. Ohrhörervorrichtung nach Anspruch 1, bei dem eine äußere Mikrophoneinheit (17), zum Umwandeln von äußerem Schal in ein elektrisches Signal, außerhalb des Akustik-Röhrchens (1) vorgesehen ist, und bei dem das Eingangs-Audiosignal, das Signal, das eine, zu dem von der inneren Mikrophoneinheit (6) erhaltenen Signal, entgegengesetzte Phasen aufweist, die von der äußeren Mikrophoneinheit (6) und inneren Mikrophoneinheit (17) erhaltenen Signale miteinander mittels einer Mischvorrichtung gemischt sind, und wobei die gemischten Signale der Ohrhörereinheit (3) zugeführt sind.

5. Ohrhörereinrichtung mit einem Akustik-Röhrchen (31), das einen Innendurchmesser (w) aufweist, der im wesentlichen mit dem äußeren Gehörgang (A) identisch ist, und das an einem Ende mit einem Ohr-Anschlußabschnitt (34) und am anderen Ende mit einem nicht-reflektierenden Akustikanschluß (35) versehen ist, einer äußeren Mikrophoneinheit (37), die außerhalb des Akustik-Röhrchens (31) angeordnet ist, um den äußeren Schal in ein elektrisches Signal umzuwandeln, einer Ohrhörereinheit (33) und einer inneren Mikrophoneinheit (36), die am Umfang des Akustik-Röhrchens (31) nahe beieinander angeordnet sind, so daß die Vibrationsscheiben der Einheiten in das Röhrchen (31) gerichtet sind, und einer veränderbaren Mischverhältniseinrichtung (38), um das Eingangs-Audiosignal, das Signal, das durch die Phasenumkehr des von der inneren Mikrophoneinheit (36) erhaltene Signal und das von der äußeren Mikrophoneinheit (37) erhaltene Signal miteinander in einem bestimmten Verhältnis zu mischen, wobei das Ausgangssignal der Mischeinrichtung (38) der Ohrhörereinheit (33) zugeführt wird.

6. Ohrhörervorrichtung nach Anspruch 5, bei der die Mischeinrichtung (38) eine erste veränderliche Verstärkungssteuerschaltung (40), zum variablen Verändern der Stärke des Eingangs-Audiosignals, eine zweite variable Verstärkungssteuerschaltung (41), zum variablen Ändern der Stärke des Signals, das durch das Signal von der inneren Mikrophoneinheit (36) erhalten wird, eine dritte veränderliche Verstärkungssteuerschaltung (42), zum variablen Ändern der Stärke des von der äußeren Mikrophoneinheit (37) erhaltenen Signals, und einen Addierer (44) aufweist, um die jeweiligen Ausgangssignale von der ersten bis dritten veränderlichen Verstärkungssteuerschaltungen (40, 41, 42) jeweils zu addieren.

7. Ohrhörervorrichtung nach Anspruch 5, bei dem die Ohrhörereinheit (33) und die innere Mikrophoneinheit (36) auf der Seite des Akustik-Röhrchens (1) angeordnet sind, so daß die jeweiligen Vibrationsscheiben parallel sind.

8. Ohrhörervorrichtung nach Anspruch 7, bei dem die Seiten der Vibrationsscheiben der Ohrhörereinheit (33) und der inneren Mikrophoneinheit (36) mit der inneren Oberfläche des Akustik-Röhrchens (31) bündig sind.

9. Ohrhörervorrichtung nach Anspruch 8, bei dem das Akustik-Röhrchen (31) einen Innendurchmesser aufweist, der im wesentlichen von dem Gehörgangs-Anordnungsabschnitt (34) zum nicht-reflektierenden Anschluß (35) konstant ist.

**Revendications**

1. Dispositif d'écouteur comprenant :
   - un tube acoustique (1) possédant un diamètre interne (W) qui est pratiquement identique à celui du canal auditif externe (A) et qui est muni d'une partie de montage sur oreille (4) à une extrémité et d'une borne sans réflexion acoustique (5) à son autre extrémité; et
   - une unité d'écouteur (3) et une unité de microphone interne (6) qui sont placées au voisinage de l'oreille lorsque le dispositif est porté et qui sont montées sur la périphérie du tube acoustique (1) de façon adjacente de telle façon que des plaques vibrantes des unités (3, 6) soient tournées vers l'intérieur du tube (1), un signal obtenu par l'unité de microphone interne (6) dont la phase est inversée étant ainsi fourni à l'unité d'écouteur (3) pour réduire les bruits externes.

2. Dispositif d'écouteur selon la revendication 1, comprenant, de plus :
   - un sommateur (9) pour ajouter le signal ayant une phase opposée à celle du signal obtenu à partir de ladite unité de microphone interne (6) au signal audio d'entrée;
   - un circuit de commande (10) pour commander la caractéristique en fréquence du signal de sortie dudit sommateur (9); et
   - un circuit d'amplification (11) pour amplifier la sortie dudit circuit de commande (10) afin de fournir la sortie amplifiée à ladite unité d'écouteur (3), les fonctions de transfert M, β, A et H de ladite unité de microphone interne (6), dudit circuit de commande (10), dudit circuit d'amplification (11) et de ladite unité d'écouteur (3), qui sont représentées en fréquence, étant ainsi établies de telle façon que la condition AHMβ >> 1 soit satisfaite.

3. Dispositif d'écouteur selon la revendication 2, dans lequel la fonction de transfert A dudit circuit d'amplification (11) est prise variable de telle façon que la condition AHMβ >> 1 soit satisfaite.

4. Dispositif d'écouteur selon la revendication 1, dans lequel une unité de microphone externe (17) pour la conversion du son externe en un signal électrique est prévue à l'extérieur dudit tube acoustique (1) et dans lequel le signal audio d'entrée, le signal ayant une phase opposée à celle du signal obtenu à partir de ladite unité de microphone interne (6), les signaux obtenus par lesdites unités de microphones externe (17) et interne (6) sont mélangés par un moyen de mélange et le signal mélangé est fourni à ladite unité d'écouteur (3).

5. Dispositif d'écouteur comprenant :
   - un tube acoustique (31) possédant un diamètre interne (W) qui est pratiquement identique à celui du canal auditif externe (A) et est muni d'une partie de montage sur oreille (34) à une extrémité et d'une borne sans réflexion acoustique (35) à son autre extrémité;

   - une unité de microphone externe (37) placée à l'extérieur dudit tube acoustique (31) pour la conversion du son externe en un signal électrique;
   - une unité d'écouteur (33) et une unité de microphone interne (36) qui sont montées sur la périphérie du tube acoustique (31), de façon adjacente, de telle façon que des plaques vibrantes des unités soient tournées vers l'intérieur du tube (31); et
   - un moyen de rapport variable de mélange (38) pour mélanger le signal audio d'entrée, le signal obtenu par l'inversion de phase du signal obtenu à partir de ladite unité de microphone interne (36) et le signal obtenu à partir de l'unité de microphone externe (37) selon un rapport désiré, le signal de sortie dudit moyen de mélange (38) étant fourni à ladite unité d'écouteur (33).

6. Unité d'écouteur selon la revendication 5, dans laquelle ledit moyen de mélange (38) comprend :
   - un premier circuit de commande à gain variable (40) pour modifier, de façon variable, le gain du signal audio d'entrée;
   - un second circuit de commande à gain variable (41) pour modifier, de façon variable, le gain du signal obtenu par inversion de phase du signal obtenu à partir de ladite unité de microphone interne (36);
   - un troisième circuit de commande à gain variable (42) pour modifier, de façon variable, le gain du signal obtenu à partir de ladite unité de microphone externe (37); et
   - un sommateur (43) pour ajouter les sorties respectives des premier à troisième circuits de commande gain variable (40, 41, 42).

7. Dispositif d'écouteur selon la revendication 5, dans lequel ladite unité d'écouteur (33) et ladite unité de microphone interne (36) sont montées sur le côté dudit tube acoustique (31) de telle façon que les plaques vibrantes respectives soient en parallèle.

8. Dispositif d'écouteur selon la revendication 7, dans lequel les côtés desdites plaques vibrantes de ladite unité d'écouteur (33) et de ladite unité de microphone interne (36) sont au niveau de la surface interne dudit tube acoustique (31).

9. Dispositif d'écouteur selon la revendication 8, dans lequel ledit tube acoustique (31) possède un diamètre interne qui est pratiquement constant de ladite partie de montage sur pavillon (34) à ladite borne sans réflexion (35).

**FIG.1**

FIG.2

**FIG.3**

FIG. 4